# EUROPEAN PATENT APPLICATION

(11) **EP 4 760 430 A1**
(43) Date of publication of application: **17.06.2026**
(21) Application number: 24873904.7
(22) Date of filing: 19.12.2024
(51) Int. Cl.: G05B 23/02

(54) **SURGICAL ROBOT TEST METHOD AND APPARATUS, DEVICE, AND STORAGE MEDIUM**

(30) Priority: 15.08.2024 CN 202411126574
(71) Applicant: Shanghai Surgipulse Robotics Co., Ltd., Shanghai 201201 (CN); Robgenix Medical Pte. Ltd., Singapore 409051 (SG)
(72) Inventor: TU, Wenbin, Shanghai 201201 (CN); ZHANG, Qianlong, Shanghai 201201 (CN); GE, Cunwang, Shanghai 201201 (CN); WU, Gang, Shanghai 201201 (CN)
(74) Representative: Westphal, Mussgnug & Partner Patentanwälte mbB
(86) International application number: PCT/CN2024/140580
(87) International publication number: WO 2026/036610

(57) **Abstract**

A surgical robot testing method, device and apparatus and a storage medium are provided herein, the method including: acquiring various surgical operation data collected by a surgical robot in performing an actual surgery; simulating a reproduced surgery performing process in a simulation environment based on the various surgical operation data; in response to a test instruction for the surgical robot during the reproduced surgery performing process, acquiring a test result corresponding to the test instruction. The present disclosure can ensure that the testing environment is more similar to real operation scenarios by using the reproduced surgery performing process as simulated, meanwhile, testing of the surgical robot in the reproduced surgery performing process is more targeted, thereby significantly improving the surgical efficiency and effectiveness of the surgical robot in use.

## Description

The present disclosure claims priority to the Chinese patent application No. CN 202411126574.3, entitled "Surgical Robot Testing Method, Device and Apparatus and Storage Medium", filed on August 15, 2024, which is incorporated herein by reference.

### TECHNICAL FIELD

The present disclosure relates to the field of computer technology, and specifically, to a surgical robot testing method, device and apparatus and a storage medium.

### BACKGROUND

At present, with the continuous development and progress of medical technology, surgical procedures are becoming more complex and sophisticated. As an important medical device, surgical robots are gradually applied in various highly difficult surgeries because they can provide precise assistance and control during surgical procedures and improve surgical efficiency and patient safety.

To ensure the operational accuracy of the surgical robots, it is generally necessary to effectively identify and resolve problems during testing of the surgical robot to ensure the normal operation of the surgical robot or avoid maloperations of the surgical robot.

However, traditional testing methods typically rely on simulators or manually created test scenarios, and thus may not fully reflect the complexity and emergencies in the actual surgical process, as a result, the operation accuracy can hardly meet the expected requirements, thereby affecting both surgical efficiency and surgical effectiveness.

### SUMMARY

Embodiments of the present disclosure at least provide a surgical robot testing method, device and apparatus and a storage medium, by which a surgical process is reproduced from the relevant data of the actual surgery performing process, to ensure that the testing environment is more similar to real operation scenarios, so that the surgical robot can deal with various complex and emergency situations in use, thereby significantly improving surgical efficiency and effectiveness.

In a first aspect, an embodiment of the present disclosure provides a surgical robot testing method, including:
acquiring various surgical operation data collected by a surgical robot in performing an actual surgery;
simulating a reproduced surgery performing process in a simulation environment based on the various surgical operation data; and
in response to a test instruction for the surgical robot during the reproduced surgery performing process, acquiring a test result corresponding to the test instruction.

Optionally, in the case where the test instruction includes a first test instruction to perform a motion control test on the surgical robot as a whole, the acquiring the test result corresponding to the test instruction includes:
acquiring motion axis parameters preset in a motion control test mode;
controlling a motion axis of the surgical robot as a whole to move according to the motion axis parameters based on the first test instruction, and displaying a motion control consequence in real time on a motion control test page;
determining whether a motor motion is correctly adjusted when the motion axis parameters are adjusted; and
determining the test result corresponding to the first test instruction based on a determined result of the adjusting.

Optionally, when the motion axis is controlled to undergo continuous motion, the determining whether the motor motion is correctly adjusted includes:
determining whether motor operating remains stable during a test on the continuous motion.

Optionally, the acquiring the motion axis parameters preset in the motion control test mode includes:
presenting the motion control test page in the motion control test mode; and
acquiring the motion axis parameters, in response to a target rotation position and a target rotation speed input by a user on the motion control test page.

Optionally, in the case where the test instruction includes a second test instruction to perform a reliability test on the surgical robot as a whole, the acquiring the test result corresponding to the test instruction includes:
acquiring various surgical operation data loaded in a reliability test mode;
controlling the surgical robot as a whole to perform the reliability test for multiple times during long-term operating based on the second test instruction, and displaying a reliability test consequence in real time on a reliability test page;
evaluating stability and thermal durability of the surgical robot by adjusting an operating speed of the surgical robot; and
determining a test result corresponding to the second test instruction based on a result of the evaluating.

Optionally, the acquiring the various surgical operation data loaded in the reliability test mode includes:
presenting the reliability test page in the reliability test mode; and
acquiring the various surgical operation data, in response to a data loading instruction triggered on the reliability test page.

Optionally, in the case where the test instruction includes a third test instruction to perform a follow-up test on the surgical robot as a whole, the acquiring the test result corresponding to the test instruction includes:
acquiring follow-up parameters preset in a follow-up test mode;
controlling a control system of the surgical robot as a whole to follow up according to the follow-up parameters based on the third test instruction, and displaying a follow-up consequence in real time on a follow-up test page;
deciding whether an actual response level of the control system meets a preset response level when the follow-up parameters are modified; and
determining a test result corresponding to the third test instruction based on a result of the deciding.

Optionally, the acquiring the follow-up parameters preset in the follow-up test mode includes:
presenting the follow-up test page in the follow-up test mode;
acquiring the follow-up parameters in response to a follow-up speed input via a speed loop menu option on the follow-up test page; or,
acquiring the follow-up parameters in response to a follow-up position input via a position loop menu option on the follow-up test page.

Optionally, in the case where the test instruction includes a fourth test instruction to perform an enabling test on the motor of the surgical robot, the acquiring the test result corresponding to the test instruction includes:
presenting a motor enabling test page in a motor enabling test mode;
in response to a trigger operation for a first enabling function on the motor enabling test page, determining whether a motor status display area is displayed as enabled; and/or,
in response to a trigger operation for a second enabling function on the motor enabling test page, determining whether the motor status display area is displayed as disenabled; and/or,
in response to a trigger operation for a third enabling function on the motor enabling test page, determining whether an enable signal is automatically sent and monitoring a starting and a stopping of the motor; and/or,
in response to a trigger operation for a fourth enabling function on the motor enabling test page, determining whether a result indication is present in a test result display area.

Optionally, in the case where the test instruction includes a fifth test instruction to perform a functional test on a pedal of the surgical robot, the acquiring the test result corresponding to the test instruction includes:
presenting a pedal function test page in a pedal function test mode;
in response to a pedal input signal from the user, determining whether a pedal response result displayed on the pedal function test page matches with an actual pedal input action; and/or,
in response to a sensitivity adjustment signal input on the pedal function test page, determining whether a pedal input action re-initiated by the sensitivity adjustment signal causes a matching pedal response result.

Optionally, in the case where the test instruction includes a sixth test instruction to perform a status test on a buzzer of the surgical robot, the acquiring the test result corresponding to the test instruction includes:
presenting a buzzer test page in a buzzer test mode;
in response to a trigger operation for a first buzzer function on the buzzer test page, determining whether the buzzer produces sound at a preset frequency; and/or,
in response to a trigger operation for a second buzzer function on the buzzer test page, determining whether an output of the buzzer is normal with different response time; and/or,
in response to a volume adjustment operation for a third buzzer function on the buzzer test page, determining whether the buzzer performs a volume adjustment operation to complete a test on volume increase or decrease.

Optionally, the test instruction includes a composite test instruction to perform a test on the surgical robot as a whole and then perform a test on each functional module of the surgical robot, and the acquiring the test result corresponding to the test instruction includes:
determining a feedback result of the surgical robot as a whole obtained by performing a test on the surgical robot as a whole;
obtaining a module feedback result from each functional module of the surgical robot when a test result of the surgical robot as a whole obtained by comparing the feedback result of the surgical robot as a whole with a preset criteria meets a preset test requirement; and
performing a verification based on the module feedback result until a normal operation criteria of the surgical robot is met.

Optionally, the method further includes:
generating a test analysis report from the test result;
outputting the test analysis report in response to an instruction for outputting the test analysis report.

Optionally, the simulating the reproduced surgery performing process in the simulation environment based on the various surgical operation data includes:
acquiring human body monitoring data collected by various medical devices;
combining the human body monitoring data collected by the medical devices and the various surgical operation data collected by the surgical robot into combined data; and
simulating the reproduced surgery performing process in the simulation environment based on the combined data.

Optionally, the simulating the reproduced surgery performing process in the simulation environment based on the various surgical operation data includes:
acquiring a pre-trained data analysis model;
analyzing the surgical operation data using the data analysis model to determine a surgical operation strategy for the surgical robot; and
simulating the reproduced surgery performing process in the simulation environment based on the surgical operation strategy.

In a second aspect, the embodiment of the present disclosure further provides a surgical robot testing device, including:
an acquisition module configured for acquiring various surgical operation data collected by a surgical robot in performing an actual surgery;
a simulation module configured for simulating a reproduced surgery performing process in a simulation environment based on the various surgical operation data; and
a testing module configured for acquiring, in response to a test instruction for the surgical robot during the reproduced surgery performing process, a test result corresponding to the test instruction.

In a third aspect, the embodiment of the present disclosure further provides an electronic device, including: a processor, a memory, and a bus, the memory storing machine-readable instructions executable by the processor, where when the electronic device is in operation, the processor is configured to communicate with the memory via the bus, and the machine-readable instructions, when executed by the processor, cause execution of the surgical robot testing method as described in any one of the first aspect and various embodiments thereof.

In a fourth aspect, an embodiment of the present disclosure further provides a computer-readable storage medium, storing a computer program that, when executed by a processor, performs the surgical robot testing method as described in any one of the first aspect and various embodiments thereof.

By using the above surgical robot testing method, device and apparatus and the storage medium, when various surgical operation data collected by the surgical robot in performing an actual surgery are obtained, the reproduced surgery performing process can be determined based on the various surgical operation data, and tests on the surgical robot as a whole and various functional modules thereof can be performed during the reproduced surgery performing process. The present disclosure can ensure that the testing environment is more similar to the real operation scenarios by using the reproduced surgery performing process as simulated, meanwhile, testing of the surgical robot in the reproduced surgery performing process is more targeted, thereby significantly improving the surgical efficiency and effectiveness of the surgical robot in use.

Other advantages of the present disclosure will be explained in more detail in combination with the following description and drawings.

It should be understood that the above summary is merely an overview of the technical solution of the present disclosure, which is provided so that the technical means of the present disclosure can be generally understood and then implemented in accordance with the contents of the specification. In order to make the above and other purposes, features and advantages of the present disclosure more apparent, the following examples are provided to illustrate the specific embodiments of the present disclosure.

### BRIEF DESCRIPTION OF DRAWINGS

In order to more clearly illustrate technical solutions according to embodiments of the present disclosure, drawings used in the embodiment are briefly introduced below. These drawings, which are incorporated herein and constitute a part of the specification, illustrate embodiments that comply with the present disclosure and are used together with the specification to illustrate the technical solutions of the present disclosure. It should be understood that the drawings only illustrate certain embodiments of the present disclosure and should not be interpreted as limiting the scope of protection. For those skilled in the art, other relevant drawings can also be derived from these drawings without creative labor. Moreover, the same reference numerals represent the same components throughout the drawings, in which:
FIG. 1 illustrates a flow chart of a surgical robot testing method provided in an embodiment of the present disclosure;
FIG. 2 illustrates a schematic diagram of displaying a motion control test page in the surgical robot testing method provided in an embodiment of the present disclosure;
FIG. 3 illustrates a schematic diagram of displaying an apparatus reliability test page in the surgical robot testing method provided in an embodiment of the present disclosure;
FIG. 4 (a) illustrates a schematic diagram of displaying a follow-up test page in the surgical robot testing method provided in an embodiment of the present disclosure;
FIG. 4 (b) illustrates another schematic diagram of displaying a follow-up test page in the surgical robot testing method provided in an embodiment of the present disclosure;
FIG. 5 illustrates a schematic diagram of displaying a motor enabling test page in the surgical robot testing method provided in an embodiment of the present disclosure;
FIG. 6 illustrates a schematic diagram of displaying a pedal function test page in the surgical robot testing method provided in an embodiment of the present disclosure;
FIG. 7 illustrates a schematic diagram of displaying a buzzer test page in the surgical robot testing method provided in an embodiment of the present disclosure;
FIG. 8 illustrates a schematic diagram of displaying a motor test page in the surgical robot testing method provided in an embodiment of the present disclosure;
FIG. 9 illustrates a schematic diagram of a surgical robot testing device provided in an embodiment of the present disclosure; and
FIG. 10 illustrates a schematic diagram of an electronic device provided in an embodiment of the present disclosure.

### DETAILED DESCRIPTION

Exemplary embodiments of the present disclosure will be described in more detail below with reference to the accompanying drawings. Although the exemplary embodiments of the present disclosure are shown in the accompanying drawings, it should be understood that the present disclosure can be implemented in various forms and should not be limited by the embodiments described herein. On the contrary, these embodiments are provided for more thorough understanding of the present disclosure and for fully conveying the scope of the present disclosure to those skilled in the art.

In the description of the embodiments of the present disclosure, it should be understood that terms such as "including" or "having" are intended to indicate the presence of the disclosed features, numbers, steps, behaviors, components, parts, or combinations thereof in the present specification, and do not exclude the possibility of the presence of one or more other features, numbers, steps, behaviors, components, parts, or combinations thereof.

Unless otherwise specified, "/" indicates the meaning of "or". For example, "A/B" may represent A or B. The term "and/or" herein is merely a way of describing the relationship between associated objects, indicating that there may be three possible relationships, for example, "A and/or B" may represent A existing alone, A and B existing simultaneously, or B existing alone.

The terms "first," "second," and the like are used merely for the convenience of description to distinguish identical or similar technical features and should not be interpreted as indicating or implying the relative importance or quantity of these technical features. Therefore, the features defined by "first", "second" and the like may explicitly or implicitly include one or more of these features. In the description of the embodiments of the present disclosure, unless otherwise specified, the term "plurality of" means two or more than two.

It has been found through research that in order to ensure the operational accuracy of surgical robots, it is generally necessary to effectively identify and resolve problems during testing of the surgical robot to ensure the normal operation of the surgical robot or avoid maloperations of the surgical robot.

However, the traditional testing methods typically rely on simulators or manually created test scenarios, and thus may not fully reflect the complexity and emergencies in the actual surgical process, as a result, the operation accuracy can hardly meet the expected requirements, thereby affecting both surgical efficiency and surgical effectiveness.

In order to at least partially solve one or more of the above problems and other potential problems, the present disclosure provides a surgical robot testing method, device and apparatus and a storage medium, by which the surgical process is reproduced from the relevant data of the actual surgery performing process, to ensure that the testing environment is more similar to real operation scenarios, so that the surgical robot can deal with various complex and emergency situations in use, thereby significantly improving surgical efficiency and effectiveness.

For ease of understanding the embodiment, a surgical robot testing method disclosed in the embodiment of the present disclosure is first introduced in detail. An execution subject of the surgical robot testing method provided in the embodiment of the present disclosure is generally an electronic device with certain computing capabilities, which includes, for example, a terminal device, or a server or other processing devices. The terminal device may be, for example, a User Equipment (UE), a mobile device, a Personal Digital Assistant (PDA), a handheld device, or a computing device, and said other processing devices may be, for example, a host device of a surgical robot. In some possible implementations, the surgical robot testing method can be implemented by a processor invoking computer-readable instructions stored in a memory.

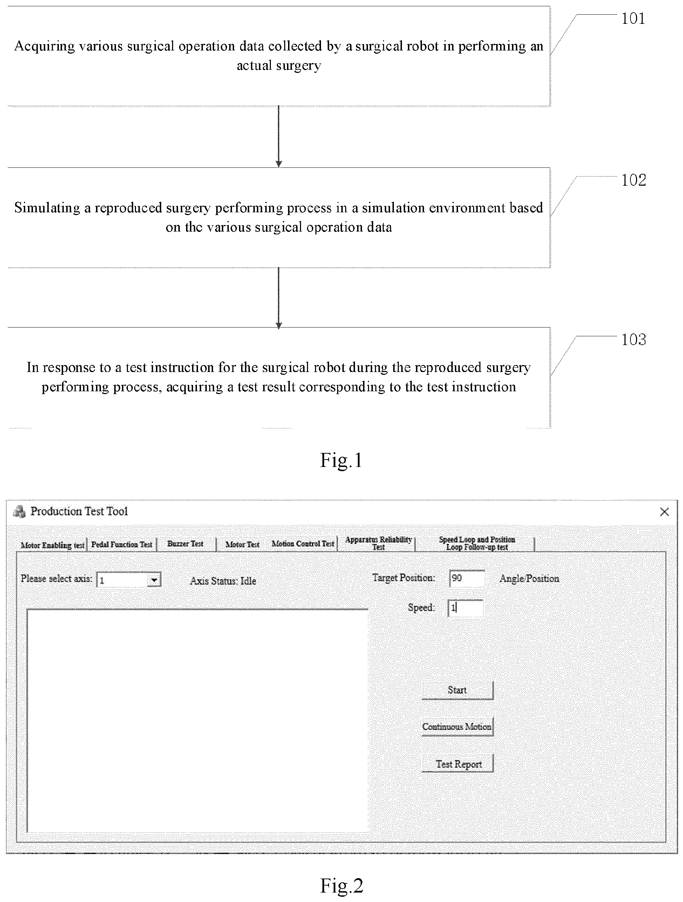

Referring to FIG. 1, which illustrates a flowchart of a surgical robot testing method provided in an embodiment of the present disclosure, and the method includes below steps S101~S103:
S101: acquiring various surgical operation data collected by a surgical robot in performing an actual surgery;
S102: simulating a reproduced surgery performing process in a simulation environment based on the various surgical operation data; and
S103: in response to a test instruction for the surgical robot during the reproduced surgery performing process, acquiring a test result corresponding to the test instruction.

For ease of understanding the surgical robot testing method provided in the embodiment of the present disclosure, an application scenario of the method is briefly described below. The surgical robot testing method may be mainly applied to various surgical procedures performed by the surgical robot, such as tumor removal surgery and cosmetic surgery, and may be also applied to remote surgical scenarios with radiotherapy effects, without specific limitations.

Considering that the related technologies typically rely on simulators or manually created test scenarios for testing and thus may not fully reflect the complexity and emergencies of the actual surgical process, an embodiment of the present disclosure provides a data-driven testing method, by which testing can be dynamically performed according to surgical operation data and more subtle operation details and abnormal situations can be captured. In addition, the surgical process is reproduced from the actual surgical history data to ensure that the test environment is more similar to the real operation scenario.

The various surgical operation data herein refer to various relevant data collected by the surgical robot during the actual execution of surgery in history, including but not limited to surgical steps, robot motion trajectories, operational force feedback and other information.

Based on the above various surgical operation data, a reproduced surgery performing process can be simulated, that is, it can be determined how the surgical robot performs the corresponding surgical operations to complete the entire surgical process. Since the simulated reproduced surgery performing process herein is determined from the various surgical operation data generated in history, the order of surgical operations, the use of surgical instruments, operational force feedback, etc., can be reproduced, thus the whole simulated process is more realistic and meaningful.

In this way, anomalies potentially occurring during a surgical process may be captured and analyzed by test operations actually performed on the surgical robot, meanwhile, the testing plan can be dynamically adjusted herein to ensure that all possible operation scenarios are covered, thereby significantly improving surgical efficiency and effectiveness of the surgical robot.

In addition, since the testing process herein is aimed at anomalies and faults found in the surgical process per se, the cause of the fault can be figured out through in-depth analysis, so that the surgical robot can be optimized and improved. Meanwhile, reperforming the reproduced surgical process by the surgical robot is enough to verify the optimization effect, thereby ensuring that the problems are completely solved, so that the surgical robot is more practical.

The surgical robot testing method provided in the embodiment of the present disclosure can adopt various testing manners, for example, the surgical robot and its various functional modules can be tested simultaneously, or the test for the surgical robot as a whole can be performed and followed by the test for each module of the surgical robot, or other testing manners can be adopted, which is not specifically limited in the embodiments of the present disclosure. Hereinafter, in most cases, tests such as a motion control test of motion axes, a reliability test of the surgical robot, a speed or position follow-up test are initially performed on the surgical robot, and then tests are performed on various functional modules of the surgical robot, such as a motor, a pedal, and a buzzer.

In an actual application, assembly workers can connect a surgical robot subsystem composed of functional modules or the surgical robot as a whole to a test and inspection system which may be an upper computer device of the surgical robot, and prepare for inspection. Here, a test software is started to simulate test instructions, and feedback signals from the surgical robot subsystem or the surgical robot are recorded and analyzed. After comparing and verifying the feedback results with the expected criteria, whether the verification passes or not is shown on a test page.

Specifically, after the expected number of test samples are accomplished, detailed test inputs and output feedbacks can be obtained through a report exporting function and serve as a basis for system testing or troubleshooting. After adjustments of the system, the verification is carried out again by the test software until the test results meet the normal operation criteria and then a record is made.

It should be noted that the actual testing process may be performed in an automated data-driven testing manner, for example, in the above testing order, thereby greatly shortening the testing cycle and improving product development efficiency. Meanwhile, testing efficiency can be greatly improved and errors potentially caused by manual operations may be reduced by automated reproduction and data analysis of the surgical process.

The test scheme provided in the embodiment of the present disclosure includes but is not limited to the tests on the surgical robot as a whole and various functional modules therein. In the embodiment of the present disclosure, the above test functions can be integrated into and implemented by a production test tool. Next, the corresponding test process will be specifically described for different test subjects.

In a first aspect, in the case where the test instructions include a first test instruction to perform a motion control test on the surgical robot as a whole, a test result corresponding to the test instruction is acquired by the following steps including:
Step 1: acquiring motion axis parameters preset in a motion control test mode;
Step 2: controlling a motion axis of the surgical robot as a whole to move according to the motion axis parameters based on the first test instruction, and displaying a motion control consequence in real time on a motion control test page;
Step 3: determining whether a motor motion is correctly adjusted when the motion axis parameters are adjusted; and
Step 4: determining the test result corresponding to the first test instruction based on a determined result of the adjusting.

As shown in FIG. 2 which is a schematic diagram of a motion control test page displayed by a production test tool provided in an embodiment of the present disclosure, on the motion control test page, a test on the motion axis can be initiated based on the first test instruction. Specifically, the test can be initiated by triggering the "Start" button, and once the "Start" button is triggered, the motion axis is moved according to the prescribed motion axis parameters, and at this time, the motion control consequence can be displayed in real time.

The motion axis parameters may be determined from a target rotation position and a target rotation speed which are entered by a user on the motion control test page presented in the motion control test mode.

Here, the motion axis parameters may also be adjusted to determine whether the coordination between the motion axis and the motor is completed. In addition, in the embodiment of the present disclosure, a continuous motion test may be performed on the motion axis to determine whether the motor operating remains stable.

In combination with the motion control test page shown in FIG. 2, the test on the motion axis can be implemented in actual applications by the following steps of:
1) presenting a motion control test page;
2) checking the current status of motion axis, to confirm that the axis status is displayed as idle;
3) setting a target position, i.e. setting the required target rotation angle or distance on the motion control test page by using an input box;
4) setting a target speed, i.e. setting an axis motion speed on the motion control test page;
5) starting motion, i.e. starting motion of the axis according to the set parameters by pressing the "Start" button;
6) monitoring the motion of the axis, i.e. observing in real time whether the axis moves according to the set parameters by using the real-time display area on the motion control test page;
7) adjusting motion parameters, to observe whether the motor motion is adjusted correctly when the target position and/or speed is changed;
8) setting a continuous motion mode, to observe the axis motion between the target position and the starting position by click the "Continuous Motion" button;
9) checking stability, i.e. performing a continuous motion test on the motor to observe whether the motor remains stable during long-term operating; and
10) exporting a test report, i.e. clicking the "Test Report" button to export the test report.

It should be noted that the above steps (1) to (10) may be executed sequentially or non-sequentially, which is not specifically limited thereto. In order to ensure the integrity of the motion control test as much as possible, these steps are executed in sequence herein.

In a second aspect, in the case where the test instruction includes a second test instruction to perform a reliability test on the surgical robot as a whole, the test result corresponding to the test instruction is acquired according to the following steps:
Step 1, acquiring various surgical operation data loaded in a reliability test mode;
Step 2, controlling the surgical robot as a whole to perform the reliability test for multiple times during long-term operating based on the second test instruction, and displaying a reliability test consequence in real time on a reliability test page;
Step 3, evaluating stability and thermal durability of the surgical robot by adjusting an operating speed of the surgical robot; and
Step 4, determining a test result corresponding to the second test instruction based on a result of the evaluating.

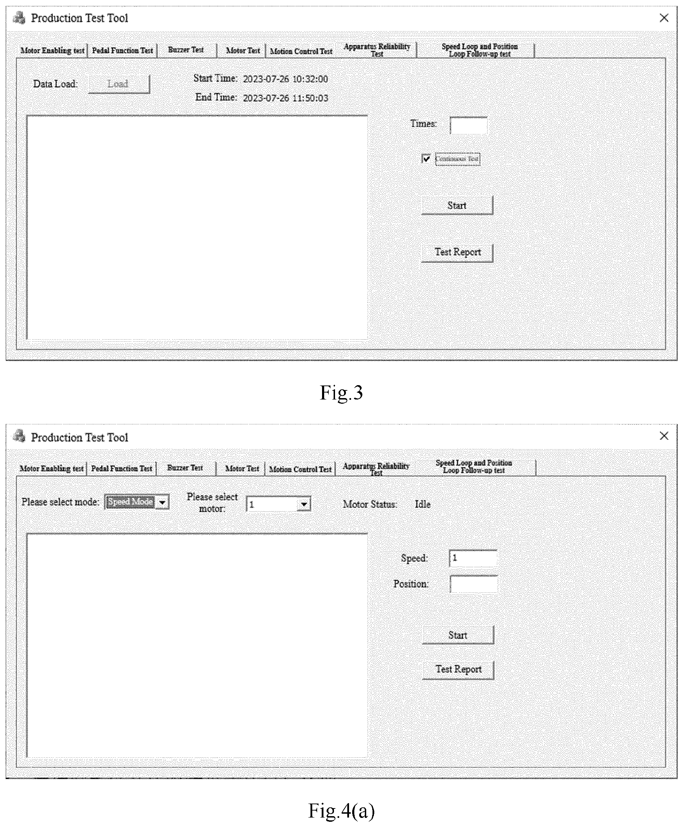

As shown in FIG. 3, which is a schematic diagram of a reliability test page displayed by a production test tool provided in an embodiment of the present disclosure. On the reliability test page, a test on the reliability of the device can be initiated by the second test instruction, specifically, by triggering the "Start" button, and once the "Start" button is triggered, a reliability test will be performed based on the loaded various surgical operation data, and then the reliability test consequence can be displayed in real time.

The various surgical operation data loaded in the reliability test mode may be the data loaded by the data loading instruction triggered on the presented reliability test page in the reliability test mode.

Here, in addition to determining the test results of the reliability test for multiple times of the surgical robot during long-term operating, the stability and thermal durability of the surgical robot can be evaluated by adjusting the operating speed of the surgical robot.

In combination with the reliability test page shown in FIG. 3, the test on device reliability can be implemented in actual application by the following steps of:
1) presenting a reliability test page;
2) checking the current device status, to confirm that the device status is displayed as idle;
3) loading data, to load historical surgical operation data by clicking the "Load" button;
4) starting a reliability test by pressing the "Start" button, to start long-term operating and repeatedly testing of the device;
5) observing the continuous operating, to observe the real-time performance of the device during the long-term operating by using the real-time display area on the reliability test page;
6) modifying the device speed, to adjust the operating speed of the device according to the test requirements;
7) testing on continuous operating, by operating the device for a long time and observing its stability under different parameters;
8) recording abnormalities, i.e., if the device is suffered from abnormalities, recording details of the abnormalities in time;
9) pausing the test, i.e. after the device operates continuously for a period of time, pausing the test to evaluate the thermal durability of the device;
10) resuming the test, i.e., resuming the test by continuing to operate the device for a long time; and
11) exporting a test report, i.e., clicking the "Test Report" button to export the test report.

It should be noted that the above steps (1) to (11) may be executed sequentially or non-sequentially, which is not specifically limited thereto. In order to ensure the completeness of the reliability test as much as possible, these steps are executed in sequence herein.

In a third aspect, in the case where the test instruction includes a third test instruction to perform a follow-up test on the surgical robot as a whole, the test result corresponding to the test instruction is acquired according to the following steps:
Step 1, acquiring follow-up parameters preset in a follow-up test mode;
Step 2, controlling a control system of the surgical robot as a whole to follow up according to the follow-up parameters based on the third test instruction, and displaying a follow-up consequence in real time on a follow-up test page;
Step 3, deciding whether an actual response level of the control system meets a preset response level when the follow-up parameters are modified; and
Step 4, determining a test result corresponding to the third test instruction based on a result of the deciding.

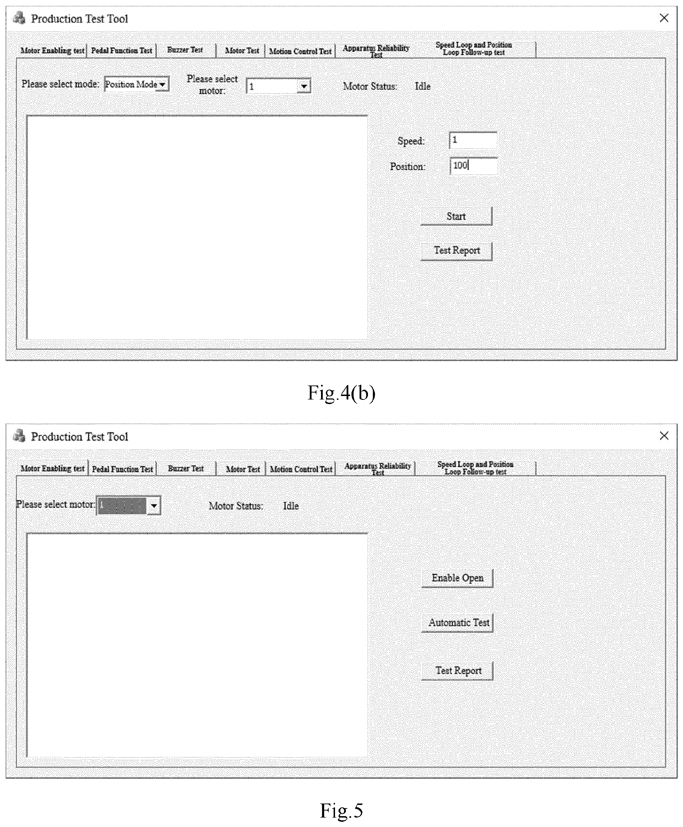

As shown in FIG. 4 (a) or FIG. 4 (b), which is a schematic diagram of a follow-up test page displayed by the production test tool provided in an embodiment of the present disclosure. On the follow-up test page, a test on the follow-up consequence of the control system can be initiated based on the third test instruction, specifically, by triggering the "Start" button, and once the "Start" button is triggered, the control system of the surgical robot will follow according to the preset follow-up parameters, and then the follow-up consequence can be displayed in real time.

The follow-up parameters can be determined based on the follow-up speed input by the user via a speed loop menu option on the follow-up test page or a follow-up position input by the user via a position loop menu option on the follow-up test page after the follow-up test page is presented in the follow-up test mode.

Here, the follow-up parameters may also be adjusted to determine whether the actual response of the control system is comparable to the preset response level.

In combination with the follow-up test page shown in FIG. 4 (a), the speed follow-up test for the control system can be implemented in actual application by the following steps of:
1) presenting a follow-up test page;
2) selecting a test mode by selecting "Speed Mode" in the menu;
3) checking status of the control system, to confirm that the control motor status is displayed as idle;
4) setting a target speed, i.e., setting the required target speed by using the provided input box;
5) start control by press the "Start" button, so that the control system starts to follow up the set target speed;
6) monitoring the system response, i.e., observing whether the control system can accurately follow the target speed, by using the real-time display area on the follow-up test page;
7) verifying speed accuracy, i.e., checking whether the actual motion speed of the device matches the target speed;
8) adjusting the target speed, to observe the response and adjustment ability of the control system when the target speed is changed;
9) testing on continuous follow-up, to observe whether the control system can operate continuously and stably by setting a test mode for continuous follow-up test mode; and
10) exporting a test report, i.e. clicking the "Test Report" button to export the test report.

It should be noted that the above steps (1) to (10) may be executed sequentially or non-sequentially, which is not specifically limited thereto. In order to ensure the completeness of the test as much as possible, these steps are executed in sequence herein.

In combination with the follow-up test page shown in FIG. 4 (b), the position follow-up test for the control system can be implemented in actual application by the following steps of:
1) presenting a follow-up test page;
2) selecting a test mode by selecting "Position Mode" in the menu;
3) checking status of the control system, to confirm that the control motor status is displayed as idle;
4) setting a target position, i.e. setting the desired target position by using the provided input box;
5) starting control by press the "Start" button, so that the control system starts to follow up the set target position;
6) monitoring the system response, i.e. observing whether the control system can accurately follow the target position, by using the real-time display area on the follow-up test page;
7) verifying position accuracy, i.e. checking whether the actual position of the device is under control and accurately matches the target position;
8) adjusting the target position, to observe the response and adjustment ability of the control system when the target position is changed;
9) testing on continuous follow-up, to observe whether the control system can operating continuously and stably by setting a continuous follow-up test mode; and
10) exporting a test report by clicking the "Test Report" button to export the test report.

Similarly, the above steps (1) to (10) can also be executed sequentially or non-sequentially, which is not specifically limited thereto. In order to ensure the completeness of the test as much as possible, these steps are executed in sequence herein.

In a fourth aspect, in the case where the test instruction includes a fourth test instruction to perform an enabling test on the motor of the surgical robot, the test result corresponding to the test instruction is acquired according to the following steps:
Step 1, presenting a motor enabling test page in a motor enabling test mode; and
Step 2, in response to a trigger operation for a first enabling function on the motor enabling test page, determining whether a motor status display area is displayed as enabled; and/or, in response to a trigger operation for a second enabling function on the motor enabling test page, determining whether the motor status display area is displayed as disenabled; and/or, in response to a trigger operation for a third enabling function on the motor enabling test page, determining whether an enable signal is automatically sent and monitoring a starting and a stopping of the motor; and/or, in response to a trigger operation for a fourth enabling function on the motor enabling test page, determining whether a result indication is present in a test result display area.

As shown in FIG. 5, which is a schematic diagram of the motor enabling test page displayed by the production test tool provided in the embodiment of the present disclosure. In the motor enabling test page, various motor enabling tests can be initiated based on the fourth test instruction, such as whether it is displayed as enabled when the motor is triggered to be turned on; also, for example, whether it is displayed as disenabled when the motor is triggered to be turned off.

In combination with the motor enabling test page shown in FIG. 5, the motor enabling test can be implemented in actual applications by the following steps of:
1) presenting a motor enabling test page;
2) selecting a motor to be tested, i.e. selecting the motor by clicking a drop-down box;
3) observing initial status of the motor, to check whether the current motor status display area shows "idle";
4) enabling, i.e. manually clicking the enable signal trigger button (i.e. the "Enabling" button) and observing and recording whether the motor status display area shows "enabled";
5) disenabling, i.e. clicking the enable signal trigger button again, and observing and recording whether the motor status display area changes to show "disenabled";
6) starting an automatic test process, i.e. clicking the "Automatic Test" button to observe whether the software automatically sends an enable signal and monitoring the starting and stopping of the motor;
7) checking test results, i.e. observing whether there is a clear result indication such as "success" or "failure" in the test result display area after the test is completed; and
8) exporting test report, i.e. clicking the "test report" button to export the test report.

It should be noted that the above steps (1) to (8) may be executed sequentially or non-sequentially, which is not specifically limited thereto. In order to ensure the completeness of the test as much as possible, these steps are executed in sequence herein.

Fifth aspect: in the case where the test instruction includes a fifth test instruction to perform a functional test on a pedal of the surgical robot, the test result corresponding to the test instruction is acquired according to the following steps:
Step 1, presenting a pedal function test page in a pedal function test mode; and
Step 2, in response to a pedal input signal from the user, determining whether a pedal response result displayed on the pedal function test page matches with an actual pedal input action; and/or, in response to a sensitivity adjustment signal input on the pedal function test page, determining whether a pedal input action re-initiated by the sensitivity adjustment signal causes a matching pedal response result.

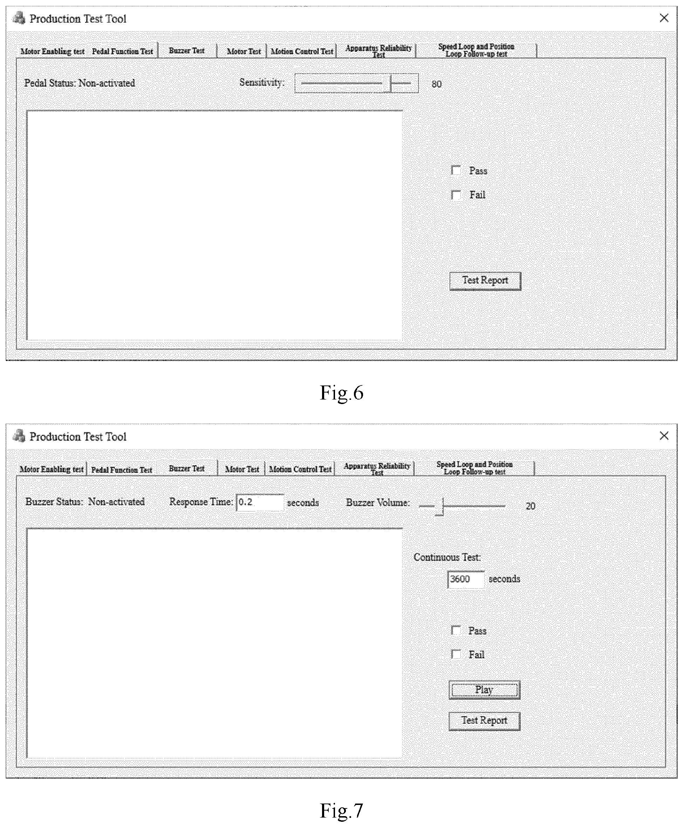

As shown in FIG. 6, which is a schematic diagram of the pedal function test page displayed by the production test tool provided in the embodiment of the present disclosure. In the pedal function test page, the pedal input signal from the user can be determined based on the fifth test instruction, and it can be determined whether the pedal response result matches with the actual pedal input action. In addition, the pedal sensitivity can be adjusted in combination with the sensitivity adjustment signal and the pedal response can be observed.

In combination with the pedal function test page shown in FIG. 6, the test for the pedal function can be implemented in actual applications by the following steps of:
1) presenting a pedal function test page.
2) checking a pedal status by confirming that the current status of the pedal is displayed as non-activated.
3) performing a pedal input by stepping on the pedal or releasing the pedal to provide an input signal.
4) observing the pedal response to make sure that the response corresponds to the input signal.
5) adjusting pedal sensitivity by using the page slider to adjust the sensitivity of the pedal signal.
6) verifying sensitivity adjustment by stepping on the pedal again and observe whether the response corresponds to the input.
7) performing continuous pedal input by performing continuous pedal operations to check whether the response of each input is correct.
8) exporting test report by clicking the "Test Report" button to export the test report.

It should be noted that the above steps (1) to (8) may be executed sequentially or non-sequentially, which is not specifically limited thereto. In order to ensure the completeness of the test as much as possible, these steps are executed in sequence herein.

Sixth aspect: in the case where the test instruction includes a sixth test instruction to perform a status test on a buzzer of the surgical robot, the test result corresponding to the test instruction is acquired according to the following steps:
Step 1, presenting a buzzer test page in a buzzer test mode;
Step 2, in response to a trigger operation for a first buzzer function on the buzzer test page, determining whether the buzzer produces sound at a preset frequency; and/or, in response to a trigger operation for a second buzzer function on the buzzer test page, determining whether an output of the buzzer is normal with different response time; and/or in response to a volume adjustment operation for a third buzzer function on the buzzer test page, determining whether the buzzer performs a volume adjustment operation to complete a test on volume increase or decrease.

As shown in FIG. 7, which is a schematic diagram of a buzzer test page displayed by the production test tool provided in an embodiment of the present disclosure. On the buzzer test page, a test for the buzzer can be initiated based on the sixth test instruction, specifically, it can be initiated by triggering the "Play" button, at this time, it can be determined whether the buzzer produces sound at a preset frequency (such as 1 second, 2 seconds, 5 seconds).

In addition, it can also be determined whether the output of the buzzer is normal with different response time, and the volume can be adjusted using the page slider to complete the test on volume increase or decrease.

In combination with the buzzer test page shown in FIG. 7, the test for the buzzer status can be implemented in actual applications by the following steps of:
1) presenting a buzzer test page.
2) checking a buzzer status by confirming that the current state of the buzzer is displayed as non-activated.
2) adjusting response time by setting the response time of the buzzer, such as 1 second.
3) triggering the buzzer by pressing the "Play" button to make the buzzer produce sound at the selected frequency.
4) evaluating sound clarity by monitoring the sound of the buzzer to confirm whether it is clear and without noise.
5) testing different response time by testing on the buzzer output of different durations in turn and confirming whether it works properly.
6) adjusting buzzer volume by using the page slider to adjust the volume.
7) verifying volume adjustment by re-triggering the buzzer to ensure that the volume increases or decreases.
8) performing a continuous play test by setting a continuous play test mode to observe whether the buzzer can continue to work normally.
9) exporting a test report by clicking the "Test Report" button to export the test report.

It should be noted that the above steps (1) to (9) may be executed sequentially or non-sequentially, which is not specifically limited thereto. In order to ensure the completeness of the test as much as possible, these steps are executed in sequence herein.

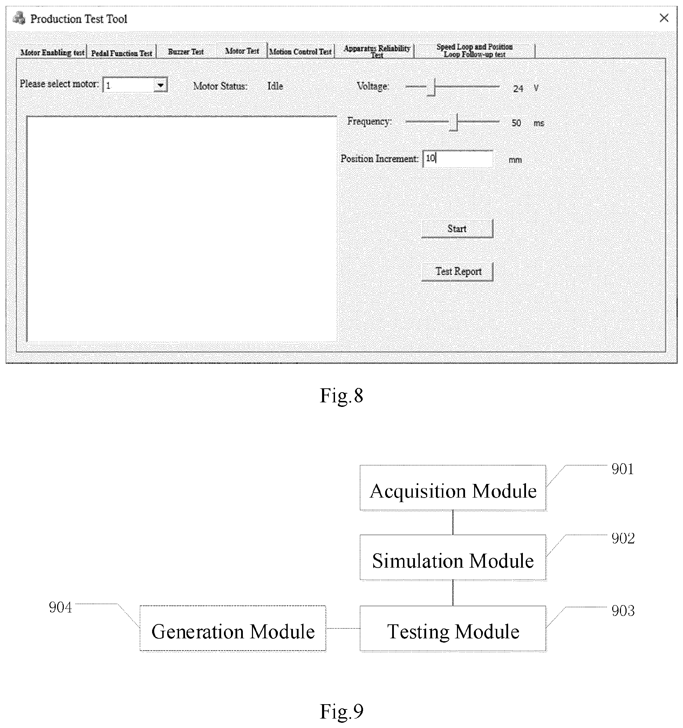

The surgical robot testing method provided in the embodiment of the present disclosure can also test on the motor of the surgical robot, as shown in FIG. 8, which can be implemented by the following steps of:
1) presenting a motor test page.
2) checking a current state of the motor by confirming that the motor status is displayed as idle.
3) setting a test voltage by using the voltage slider provided on the page to select a specific test voltage.
4) starting the motor by pressing the "Start" button to start the motor running.
5) monitoring the speed, i.e., observing whether the real-time speed of the motor corresponds to the expected speed by using a speed display area.
6) adjusting the test frequency by using the frequency slider on the page to set the frequency to be tested.
7) adjusting the position increment by using the edit box on the page to enter the position increment required for sending each command.
8) verifying motor frequency response by starting the motor again and observing its speed changes at different frequencies.
9) measuring torque by viewing the real-time torque of the motor at a given voltage and frequency in the display area.
10) performing a continuous run test by setting a duration, such as 5 minutes or 10 minutes, to see if the motor can work stably.
11) exporting a test report by clicking the "Test Report" button to export the test report.

It should be noted that the above steps (1) to (11) may be executed sequentially or non-sequentially, which is not specifically limited thereto. In order to ensure the completeness of the test as much as possible, these steps are executed in sequence herein.

It should be noted that the above various test schemes may be executed separately or sequentially based on the test needs of the user, the entire process is automatically operated, which can greatly improve the test efficiency.

In the actual test process, a test on the surgical robot as a whole can be performed and then a test on each functional module of the surgical robot can be performed, specifically, it can be achieved through the following steps including:
Step 1, determining a feedback result of the surgical robot as a whole obtained by performing a test on the surgical robot as a whole;
Step 2, obtaining a module feedback result from each functional module of the surgical robot when a test result of the surgical robot as a whole obtained by comparing the feedback result of the surgical robot as a whole with a preset criteria meets a preset test requirement; and
Step 3, performing a verification based on the module feedback result until a normal operation criteria of the surgical robot is met.

Here, when it is determined that the surgical robot as a whole meets the test requirements, a test on each functional module is performed, and based on further verification of the module feedback result, the normal operation of the surgical robot is adjusted. Here, on the one hand, the use of real surgical operation data improves the accuracy of the test, and can more effectively identify problems that may occur in the actual use of the device; on the other hand, through more rigorous and comprehensive testing, the reliability and safety of the device in actual surgery are significantly improved.

Based on the above description, it can be seen that when the test results are determined, the embodiment of the present disclosure can generate a test analysis report from the test results, and in response to the instruction for outputting the test analysis report, the test analysis report is output for the user to view.

In order to further improve the test effect of the surgical robot, the surgical robot testing method provided in the embodiment of the present disclosure can also integrate various technical means to achieve the reproduction of the relevant surgical execution process.

Firstly, the data of other medical apparatus such as patient vital signs monitoring apparatus, imaging apparatus in the operating room can be integrated to build a more comprehensive surgical process model to further improve the authenticity and comprehensiveness of the test. Specifically, the reproduced surgery performing process can be implemented according to the following steps:
Step 1, acquiring human body monitoring data collected by various medical devices;
Step 2, combining the human body monitoring data collected by the medical devices and the various surgical operation data collected by the surgical robot into combined data; and
Step 3, simulating the reproduced surgery performing process in the simulation environment based on the combined data.

Here, the human monitoring data collected by the medical apparatus can be combined to determine the actual execution of surgery that the surgical robot needs to perform, achieving the mutual integration of various data, so that the entire surgical process can be reproduced more accurately, which further enhances the effectiveness of the test.

Secondly, machine learning and artificial intelligence can be used herein to reproduce the surgical execution process, specifically by the following steps:
Step 1, acquiring a pre-trained data analysis model;
Step 2, analyzing the surgical operation data using the data analysis model to determine a surgical operation strategy for the surgical robot; and
Step 3, simulating the reproduced surgery performing process in the simulation environment based on the surgical operation strategy.

In practical applications, a large amount of surgical operation data can be analyzed to extract potential patterns and regularities in surgical operations, and optimize the operation strategy and test plan of the surgical robot.

In addition, the surgical robot testing method provided in the embodiment of the present disclosure can also implement remote testing and collaborative testing by the network to further improve the test effect. In practical applications, testers can share test data and test processes in real time at different locations to improve the efficiency and coordination of testing.

In the description of the present specification, reference terms such as such as "some possible embodiments", "some embodiments", "examples", "specific examples", or "some examples" are used to describe that the specific features, structures, materials or characteristics described in conjunction with the embodiment or example are included in at least one embodiment or example of the present disclosure, and the above terms do not necessarily represent the same embodiment or example. Moreover, the described specific features, structures, materials or characteristics can be combined in any one or more embodiments or examples in a suitable manner. In addition, the different embodiments or examples described in the present specification and the features of different embodiments or examples can be combined and combined by those skilled in the art without contradiction.

Regarding the method flowchart of the embodiment of the present disclosure, some operations are described as different steps executed in a certain order. Such a flowchart is illustrative rather than restrictive. Some steps described herein can be grouped together and performed in a single operation, or some steps can be divided into multiple sub-steps, and some steps can be performed in an order different from that shown herein. The various steps shown in the flowchart can be implemented by any circuit structure and/or tangible mechanism (for example, software running on a computer device, hardware (for example, a logical function implemented by a processor or chip), and/or any combination thereof).

Those skilled in the art can understand that in the method described in the above specific embodiments, the writing order of each step does not mean a strict execution order, and the specific execution order of each step should be determined by its function and possible internal logic.

Based on the same inventive concept, the embodiment of the present disclosure further provides a surgical robot testing device corresponding to the surgical robot testing method, since the principle of the device in the embodiment of the present disclosure to solve the problem is similar to the above surgical robot testing method in the embodiment of the present disclosure, the implementation of the device can refer to the implementation of the method, and the repetitive parts will not be repeated.

Referring to FIG. 9, which is a schematic diagram of a surgical robot testing device provided in the embodiment of the present disclosure. The above device includes: an acquisition module 901, a simulation module 902, and a testing module 903; in which,
the acquisition module 901 is configured to acquire various surgical operation data collected by a surgical robot in performing an actual surgery;
the simulation module 902 is configured to simulate a reproduced surgery performing process in a simulation environment based on the various surgical operation data; and
the testing module 903 is configured to acquire, in response to a test instruction for the surgical robot during the reproduced surgery performing process, a test result corresponding to the test instruction.

By using the above surgical robot testing device, when various surgical operation data collected by the surgical robot in performing an actual surgery are obtained, the reproduced surgery performing process can be determined based on the various surgical operation data, and the tests on the surgical robot as a whole and various functional modules thereof can be performed during the reproduced surgery performing process. The present disclosure can ensure that the testing environment is more similar to the real operation scenarios by using the reproduced surgery performing process as simulated, meanwhile, testing of the surgical robot in the reproduced surgery performing process is more targeted, thereby significantly improving the surgical efficiency and effectiveness of the surgical robot in use.

Optionally, in the case where the test instruction includes a first test instruction to perform a motion control test on the surgical robot as a whole, the testing module 903 is specifically configured to acquire the test result corresponding to the test instruction according to the following steps of:
acquiring motion axis parameters preset in a motion control test mode;
controlling a motion axis of the surgical robot as a whole to move according to the motion axis parameters based on the first test instruction, and displaying a motion control consequence in real time on a motion control test page;
determining whether a motor motion is correctly adjusted when the motion axis parameters are adjusted; and
determining the test result corresponding to the first test instruction based on a determined result of the adjusting.

Optionally, when the motion axis is controlled to undergo continuous motion, the testing module 903 is specifically configured to determine whether the motor motion is correctly adjusted according to a step of:
determining whether motor operating remains stable during a test on the continuous motion.

Optionally, the testing module 903 is specifically configured to acquire the motion axis parameters preset in the motion control test mode according to steps of:
presenting the motion control test page in the motion control test mode; and
acquiring the motion axis parameters, in response to a target rotation position and a target rotation speed input by a user on the motion control test page.

Optionally, in the case where the test instruction includes a second test instruction to perform a reliability test on the surgical robot as a whole, the testing module 903 is specifically configured to acquire the test result corresponding to the test instruction according to the following steps of:
acquiring various surgical operation data loaded in a reliability test mode;
controlling the surgical robot as a whole to perform the reliability test for multiple times during long-term operating based on the second test instruction, and displaying a reliability test consequence in real time on a reliability test page;
evaluating stability and thermal durability of the surgical robot by adjusting an operating speed of the surgical robot; and
determining a test result corresponding to the second test instruction based on a result of the evaluating.

Optionally, the testing module 903 is specifically configured to acquire the various surgical operation data loaded in the reliability test mode according to the following steps of:
presenting the reliability test page in the reliability test mode; and
acquiring the various surgical operation data, in response to a data loading instruction triggered on the reliability test page.

Optionally, in the case where the test instruction includes a third test instruction to perform a follow-up test on the surgical robot as a whole, the testing module 903 is specifically configured to acquire the test result corresponding to the test instruction according to the following steps of:
acquiring follow-up parameters preset in a follow-up test mode;
controlling a control system of the surgical robot as a whole to follow up according to the follow-up parameters based on the third test instruction, and displaying a follow-up consequence in real time on a follow-up test page;
deciding whether an actual response level of the control system meets a preset response level when the follow-up parameters are modified; and
determining a test result corresponding to the third test instruction based on a result of the deciding.

Optionally, the testing module 903 is specifically configured to acquire the follow-up parameters preset in the follow-up test mode according to the following steps of:
presenting the follow-up test page in the follow-up test mode;
acquiring the follow-up parameters in response to a follow-up speed input via a speed loop menu option on the follow-up test page; or,
acquiring the follow-up parameters in response to a follow-up position input via a position loop menu option on the follow-up test page.

Optionally, in the case where the test instruction includes a fourth test instruction to perform an enabling test on the motor of the surgical robot, the testing module 903 is specifically configured to acquire the test result corresponding to the test instruction according to the following steps of:
presenting a motor enabling test page in a motor enabling test mode;
in response to a trigger operation for a first enabling function on the motor enabling test page, determining whether a motor status display area is displayed as enabled; and/or,
in response to a trigger operation for a second enabling function on the motor enabling test page, determining whether the motor status display area is displayed as disenabled; and/or,
in response to a trigger operation for a third enabling function on the motor enabling test page, determining whether an enable signal is automatically sent and monitoring a starting and a stopping of the motor; and/or,
in response to a trigger operation for a fourth enabling function on the motor enabling test page, determining whether a result indication is present in a test result display area.

Optionally, in the case where the test instruction includes a fifth test instruction to perform a functional test on a pedal of the surgical robot, the testing module 903 is specifically configured to acquire the test result corresponding to the test instruction according to the following steps of:
presenting a pedal function test page in a pedal function test mode;
in response to a pedal input signal from the user, determining whether a pedal response result displayed on the pedal function test page matches with an actual pedal input action; and/or,
in response to a sensitivity adjustment signal input on the pedal function test page, determining whether a pedal input action re-initiated by the sensitivity adjustment signal causes a matching pedal response result.

Optionally, in the case where the test instruction includes a sixth test instruction to perform a status test on a buzzer of the surgical robot, the testing module 903 is specifically configured to acquire the test result corresponding to the test instruction according to the following steps of:
presenting a buzzer test page in a buzzer test mode;
in response to a trigger operation for a first buzzer function on the buzzer test page, determining whether the buzzer produces sound at a preset frequency; and/or,
in response to a trigger operation for a second buzzer function on the buzzer test page, determining whether an output of the buzzer is normal with different response time; and/or,
in response to a volume adjustment operation for a third buzzer function on the buzzer test page, determining whether the buzzer performs a volume adjustment operation to complete a test on volume increase or decrease.

Optionally, in the case where the test instruction includes a composite test instruction to perform a test on the surgical robot as a whole and then perform a test on each functional module of the surgical robot, the testing module 903 is specifically configured to acquire the test result corresponding to the test instruction according to the following steps of:
determining a feedback result of the surgical robot as a whole obtained by performing a test on the surgical robot as a whole;
obtaining a module feedback result from each functional module of the surgical robot when a test result of the surgical robot as a whole obtained by comparing the feedback result of the surgical robot as a whole with a preset criteria meets a preset test requirement; and
performing a verification based on the module feedback result until a normal operation criterion of the surgical robot is met.

Optionally, the device further includes:
generation module 904, configured to generate a test analysis report from the test result; and output the test analysis report in response to an instruction for outputting the test analysis report.

Optionally, the simulation module 902 is specifically configured to simulate the reproduced surgery performing process in the simulation environment based on various surgical operation data according to the following steps of:
acquiring human body monitoring data collected by various medical devices;
combining the human body monitoring data collected by the medical devices and the various surgical operation data collected by the surgical robot into combined data; and
simulating the reproduced surgery performing process in the simulation environment based on the combined data.

Optionally, the simulation module 902 is specifically configured to simulate the reproduced surgery performing process in the simulation environment based on various surgical operation data according to the following steps of:
acquiring a pre-trained data analysis model;
analyzing the surgical operation data using the data analysis model to determine a surgical operation strategy for the surgical robot; and
simulating the reproduced surgery performing process in the simulation environment based on the surgical operation strategy.

It should be noted that the device in the embodiment of the present disclosure can implement each process of the embodiments of the above methods and achieve the same effects and functions, which will not be repeated herein.

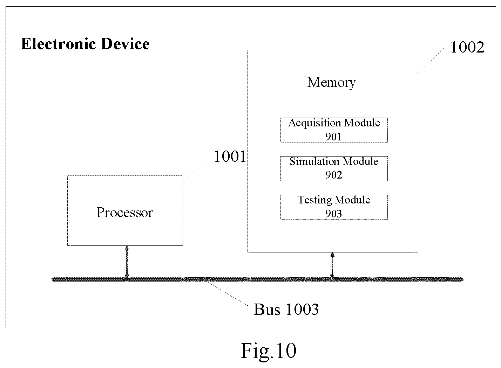

The embodiment of the present disclosure further provides an electronic device, as shown in FIG. 10, which is a schematic diagram of the electronic device structure provided in the embodiment of the present disclosure, including: a processor 1001, a memory 1002, and a bus 1003. The memory 1002 stores machine-readable instructions executable by the processor 1001 (for example, the execution instructions corresponding to the acquisition module 901, the simulation module 902, and the testing module 903 in the device in FIG. 9), the processor 1001 is configured to communicate with the memory 1002 via the bus 1003 when the electronic device is in operation, and the machine-readable instructions, when executed by the processor 1001, cause execution of the following steps of:
acquiring various surgical operation data collected by a surgical robot in performing an actual surgery;
simulating a reproduced surgery performing process in a simulation environment based on the various surgical operation data; and
in response to a test instruction for the surgical robot during the reproduced surgery performing process, acquiring a test result corresponding to the test instruction.

The embodiment of the present disclosure further provides a computer-readable storage medium, storing a computer program that, when executed by a processor, performs the surgical robot testing method described in the above embodiments. The storage medium can be a volatile or non-volatile computer-readable storage medium.

The embodiments of the present disclosure further provide a computer program product, including a computer program, which carries program codes, and the instructions included in the program codes are configured for performing the method provided in the above embodiments, which is specifically described in the above method embodiments and will not be repeated herein.

The above computer program product can be implemented specifically by hardware, software or a combination thereof. In an optional embodiment, the computer program product is specifically embodied as a computer storage medium, and in another optional embodiment, the computer program product is specifically embodied as a software product, such as a software development kit (SDK) and the like.

Each embodiment in the present disclosure is described in a progressive manner, and the same or similar parts between the various embodiments can be referenced to each other, and each embodiment focuses on the differences from other embodiments. In particular, for the apparatus, device and computer-readable storage medium implementation, since they are basically similar to the method implementation, their descriptions are simplified, and the relevant parts can refer to the partial description of the method implementation.

The apparatus, device and computer-readable storage medium provided in the embodiment of the present disclosure each correspond to the method, and thus likewise have similar beneficial technical effects as the corresponding method. Since the beneficial technical effects of the method have been described in detail above, the beneficial technical effects of the apparatus, device, and computer-readable storage medium will not be repeated herein.

Those skilled in the art should understand that the embodiments of the present disclosure can be implemented in the form of methods and apparatus (devices or systems), or computer-readable storage media. Therefore, the present disclosure can utilize a full hardware implementation, a full software implementation, or a combination of software and hardware. Moreover, the present disclosure can utilize the form of a computer-readable storage medium implemented on one or more computer-usable storage media (including but not limited to disk storage, read-only optical disk storage (CD-ROM), optical storage) containing computer-usable program codes.

The present disclosure is described with reference to the flowchart and/or block diagram of the method, apparatus (device or system) and computer-readable storage medium according to the embodiments of the present disclosure. It should be understood that each process and/or block in the flowchart and/or block diagram, as well as the combination of processes and/or blocks in the flowchart and/or block diagram, can be implemented by computer program instructions. These computer program instructions can be provided to a processor of a general-purpose computer, a special-purpose computer, an embedded processor or other programmable data processing device to produce a machine, so that the instructions executed by the processor of the computer or other programmable data processing device produce means for implementing the functions specified in one or more processes in the flowchart and/or one or more blocks in the block diagram.

These computer program instructions can also be stored in a computer-readable memory that can guide a computer or other programmable data processing device to work in a specific manner, so that the instructions stored in the computer-readable memory produce a product including an instruction device, where the functions specified in one or more processes in the flowchart and/or one or more blocks in the block diagram can be implemented by the instruction device.

These computer program instructions can also be loaded onto a computer or other programmable data processing device, so that a series of operation steps are performed on the computer or other programmable device to produce a computer-implemented process, so that the instructions executed on the computer or other programmable device provide steps for implementing the functions specified in one or more processes in the flowchart and/or one or more blocks in the block diagram.

In a typical configuration, a computing device includes one or more processors (CPUs), an input/output interface, a network interface, and a memory.

Memory may include forms of non-permanent storage in computer-readable medium, such as random access memory (RAM) and/or non-volatile memory, such as read-only memory (ROM) or flash memory (Flash RAM). Memory is an example of a computer-readable medium.

Computer-readable media include permanent and non-permanent, removable and non-removable media, and can be implemented by any method or technology to store information. Information can be computer-readable instructions, data structures, modules of programs, or other data. Examples of computer-readable storage media include, but are not limited to, phase change memory (PRAM), static random access memory (SRAM), dynamic random access memory (DRAM), other types of random access memory, read-only memory, electrically erasable programmable read-only memory (EEPROM), flash memory or other memory technology, CD-ROM, digital versatile disc (DVD) or other optical storage, magnetic cassettes, magnetic tape magnetic disk storage or other magnetic storage devices or any other non-transmission medium that can be used to store information that can be accessed by a computing device. In addition, although the operations of the method of the present disclosure are described in a particular order in the accompanying drawings, this does not require or imply that the operations must be performed in this particular order or that all of the operations shown must be performed to achieve the desired result. In addition, some steps may be omitted, multiple steps may be combined into one step, and/or one step may be decomposed into multiple sub-steps.

Although the spirit and principle of the present disclosure have been described with reference to several specific implementation methods above, it should be understood that the present disclosure is not limited to the disclosed specific implementation methods, and the division of various aspects does not mean that the features in these aspects cannot be combined. The present disclosure is intended to cover various modifications and equivalent arrangements included within the spirit and scope of the appended claims.

## Claims

1. A surgical robot testing method, comprising:
acquiring various surgical operation data collected by a surgical robot in performing an actual surgery;
simulating a reproduced surgery performing process in a simulation environment based on the various surgical operation data; and
in response to a test instruction for the surgical robot during the reproduced surgery performing process, acquiring a test result corresponding to the test instruction.

2. The method according to claim 1, wherein, if the test instruction comprises a first test instruction to perform a motion control test on the surgical robot as a whole, the acquiring the test result corresponding to the test instruction comprises:
acquiring motion axis parameters preset in a motion control test mode;
controlling a motion axis of the surgical robot as a whole to move according to the motion axis parameters based on the first test instruction, and displaying a motion control consequence in real time on a motion control test page;
determining whether a motor motion is correctly adjusted when the motion axis parameters are adjusted; and
determining the test result corresponding to the first test instruction based on a determined result of the adjusting.

3. The method according to claim 2, wherein, when the motion axis is controlled to undergo continuous motion, the determining whether the motor motion is correctly adjusted comprises:
determining whether motor operating remains stable during a test on the continuous motion.

4. The method according to claim 2 or 3, wherein the acquiring the motion axis parameters preset in the motion control test mode comprises:
presenting the motion control test page in the motion control test mode; and
acquiring the motion axis parameters, in response to a target rotation position and a target rotation speed input by a user on the motion control test page.

5. The method according to any one of claims 1 to 4, wherein, if the test instruction comprises a second test instruction to perform a reliability test on the surgical robot as a whole, the acquiring the test result corresponding to the test instruction comprises:
acquiring various surgical operation data loaded in a reliability test mode;
controlling the surgical robot as a whole to perform the reliability test for multiple times during long-term operating based on the second test instruction, and displaying a reliability test consequence in real time on a reliability test page;
evaluating stability and thermal durability of the surgical robot by adjusting an operating speed of the surgical robot; and
determining a test result corresponding to the second test instruction based on a result of the evaluating.

6. The method according to claim 5, wherein the acquiring the various surgical operation data loaded in the reliability test mode comprises:
presenting the reliability test page in the reliability test mode; and
acquiring the various surgical operation data, in response to a data loading instruction triggered on the reliability test page.

7. The method according to any one of claims 1 to 6, wherein, if the test instruction comprises a third test instruction to perform a follow-up test on the surgical robot as a whole, the acquiring the test result corresponding to the test instruction comprises:
acquiring follow-up parameters preset in a follow-up test mode;
controlling a control system of the surgical robot as a whole to follow up according to the follow-up parameters based on the third test instruction, and displaying a follow-up consequence in real time on a follow-up test page;
deciding whether an actual response level of the control system meets a preset response level when the follow-up parameters are modified; and
determining a test result corresponding to the third test instruction based on a result of the deciding.

8. The method according to claim 7, wherein, the acquiring the follow-up parameters preset in the follow-up test mode comprises:
presenting the follow-up test page in the follow-up test mode;
acquiring the follow-up parameters in response to a follow-up speed input via a speed loop menu option on the follow-up test page; or
acquiring the follow-up parameters in response to a follow-up position input via a position loop menu option on the follow-up test page.

9. The method according to any one of claims 1 to 8, wherein, if the test instruction comprises a fourth test instruction to perform an enabling test on the motor of the surgical robot, the acquiring the test result corresponding to the test instruction comprises:
presenting a motor enabling test page in a motor enabling test mode;
in response to a trigger operation for a first enabling function on the motor enabling test page, determining whether a motor status display area is displayed as enabled; and/or
in response to a trigger operation for a second enabling function on the motor enabling test page, determining whether the motor status display area is displayed as disenabled; and/or
in response to a trigger operation for a third enabling function on the motor enabling test page, determining whether an enable signal is automatically sent and monitoring a starting and a stopping of the motor; and/or
in response to a trigger operation for a fourth enabling function on the motor enabling test page, determining whether a result indication is present in a test result display area.

10. The method according to any one of claims 1 to 9, wherein, if the test instruction comprises a fifth test instruction to perform a functional test on a pedal of the surgical robot, the acquiring the test result corresponding to the test instruction comprises:
presenting a pedal function test page in a pedal function test mode;
in response to a pedal input signal from the user, determining whether a pedal response result displayed on the pedal function test page matches with an actual pedal input action; and/or
in response to a sensitivity adjustment signal input on the pedal function test page, determining whether a pedal input action re-initiated by the sensitivity adjustment signal causes a matching pedal response result.

11. The method of any one of claims 1 to 10, wherein, if the test instruction comprises a sixth test instruction to perform a status test on a buzzer of the surgical robot, the acquiring the test result corresponding to the test instruction comprises:
presenting a buzzer test page in a buzzer test mode;
in response to a trigger operation for a first buzzer function on the buzzer test page, determining whether the buzzer produces sound at a preset frequency; and/or,
in response to a trigger operation for a second buzzer function on the buzzer test page, determining whether an output of the buzzer is normal with different response time; and/or
in response to a volume adjustment operation for a third buzzer function on the buzzer test page, determining whether the buzzer performs a volume adjustment operation to complete a test on volume increase or decrease.

12. The method according to any one of claims 1 to 11, wherein the test instruction comprises a composite test instruction to perform a test on the surgical robot as a whole and then perform a test on each functional module in the surgical robot, and the acquiring the test result corresponding to the test instruction comprises:
determining a feedback result of the surgical robot as a whole obtained by performing the test on the surgical robot as a whole;
obtaining a module feedback result from each functional module of the surgical robot when a test result of the surgical robot as a whole obtained by comparing the feedback result of the surgical robot as a whole with a preset criteria meets a preset test requirement; and
performing a verification based on the module feedback result until a normal operation criteria of the surgical robot is met.

13. The method according to any one of claims 1 to 12, wherein the method further comprises:
generating a test analysis report from the test result; and
outputting the test analysis report in response to an instruction for outputting the test analysis report.

14. The method according to any one of claims 1 to 12, wherein the simulating the reproduced surgery performing process in the simulation environment based on the various surgical operation data comprises:
acquiring human body monitoring data collected by various medical devices;
combining the human body monitoring data collected by the medical devices and the various surgical operation data collected by the surgical robot into combined data; and
simulating the reproduced surgery performing process in the simulation environment based on the combined data.

15. The method according to any one of claims 1 to 12, wherein the simulating the reproduced surgery performing process in the simulation environment based on the various surgical operation data comprises:
acquiring a pre-trained data analysis model;
analyzing the surgical operation data using the data analysis model to determine a surgical operation strategy for the surgical robot; and
simulating the reproduced surgery performing process in the simulation environment based on the surgical operation strategy.

16. A surgical robot testing device, comprising:
an acquisition module configured for acquiring various surgical operation data collected by a surgical robot in performing an actual surgery;
a simulation module configured for simulating a reproduced surgery performing process in a simulation environment based on the various surgical operation data; and
a testing module configured for acquiring, in response to a test instruction for the surgical robot during the reproduced surgery performing process, a test result corresponding to the test instruction.

17. An electronic device, comprising: a processor, a memory, and a bus, the memory storing machine-readable instructions executable by the processor, wherein when the electronic device is in operation, the processor communicates with the memory via the bus, and the machine-readable instructions, when executed by the processor, cause execution of the surgical robot testing method according to any one of claims 1 to 15.

18. A computer-readable storage medium, storing a computer program that, when executed by a processor, performs the surgical robot testing method according to any one of claims 1 to 15.
